# FASCICULE DE BREVET EUROPEEN

(11) **EP 1 336 417 B1**
(45) Date de publication et mention de la délivrance du brevet: **03.01.2007**
(21) Numéro de dépôt: 03290289.2
(22) Date de dépôt: 05.02.2003
(51) Int. Cl.: A61M 1/36, A61M 1/02, B01D 39/16, B01D 15/00, A61M 5/165

(54) **Unité de filtration comprenant des couches deleucocytantes calandrees**
Filtereinheit mit kalandrierten Schichten zur Entfernung von Leukozyten
Filter unit comprising leucocytes removing calandered layers

(30) Priorité: 13.02.2002 FR 0201776
(43) Date de publication de la demande: 20.08.2003
(73) Titulaire: MACO PHARMA, 59420 Mouvaux (FR)
(72) Inventeur: Verpoort, Thierry, 59420 Mouvaux (FR); Chollet, Stéphane, 59420 Mouvaux (FR)
(74) Mandataire: Geismar, Thierry

(56) Documents cités:
- EP-A- 0 313 348
- EP-A- 0 397 403
- EP-A- 0 526 678
- EP-A- 0 591 980
- EP-A- 0 948 993
- EP-A- 0 953 361
- EP-A- 0 976 413
- WO-A-01/74159
- WO-A-02/07854
- WO-A-98/19722

## Description

L'invention concerne une unité de filtration destinée à permettre la déleucocytation d'un fluide ainsi qu'un système à poches comprenant une telle unité de filtration.

Elle s'applique typiquement à la filtration du sang ou d'un composant sanguin ainsi qu'à la séparation et au recueil de différents constituants du sang dans le système à poches, et ce notamment en circuit clos.

On connaît déjà des unités de filtration qui comprennent une enveloppe extérieure munie d'au moins un orifice d'entrée et d'au moins un orifice de sortie entre lesquels le fluide à filtrer s'écoule suivant une direction, l'enveloppe renfermant un élément poreux comprenant un milieu de déleucocytation par adsorption et par filtration des leucocytes.

Dans de telles unités, illustrées par exemple par le document EP-A-0 526 678, il est classique d'utiliser, en tant que milieu de déleucocytation, un empilement de couches filtrantes formées d'un non-tissé poreux.

En effet, dans ce type de filtration -dite en profondeur-, la capacité du milieu filtrant à retenir les leucocytes est fonction notamment de la quantité de matière traversée par le fluide, et donc de l'épaisseur du milieu filtrant. En outre, la disposition d'une pluralité de couches fines permet d'améliorer le rendement de déleucocytation par rapport à un milieu filtrant de même épaisseur totale et qui serait formé d'une seule couche.

EP-A-948 993 montre un procédé où une feuille de polytétrafluoréthylène est pressée par calandrage pour obtenir une plus petite taille de pores.

Pour améliorer l'efficacité de ce type de filtration, c'est-à-dire augmenter la quantité de leucocytes retenue par le milieu de déleucocytation, on a donc pensé à augmenter le nombre de couches empilées.

Mais cette solution présente un certain nombre d'inconvénients.

En premier lieu, elle implique une augmentation de la taille globale du filtre ce qui, de façon générale, n'est pas souhaitable. En outre, elle conduit à une augmentation du volume mort de l'unité de filtration, c'est-à-dire de la quantité de fluide restant dans l'unité de filtration après la filtration, ce fluide étant par conséquent soit perdu soit difficilement récupérable. En particulier, dans les unités de filtration destinées à filtrer une quantité faible de fluide, cette contrainte devient rapidement rédhibitoire.

Ensuite, l'augmentation du nombre de couches provoque une diminution sensible du débit de fluide passant à travers le milieu de déleucocytation par gravité, et donc augmente d'autant le temps de filtration.

Par ailleurs, la demanderesse a constaté que, à partir d'une certaine valeur, cette augmentation n'avait plus d'effet positif notable sur la quantité de leucocytes retenue par le milieu de déleucocytation.

L'invention vise donc à remédier à ces inconvénients en proposant notamment une unité présentant une capacité améliorée et adaptable de filtration, et ce sans influer de façon néfaste sur le débit de filtration, la taille de l'unité de filtration et son volume mort. En outre, l'unité de filtration peut être intégrée dans un système à poches, notamment en circuit clos, afin de permettre, de façon simple, la séparation et le recueil de différents constituants du sang.

A cet effet, et selon un premier aspect, l'invention propose une unité de filtration destinée à permettre la déleucocytation d'un fluide tel que le sang ou un composant sanguin, du type comprenant une enveloppe extérieure munie d'au moins un orifice d'entrée et d'au moins un orifice de sortie entre lesquels le fluide à filtrer s'écoule suivant une direction, l'enveloppe renfermant un élément poreux comprenant un milieu de déleucocytation par adsorption et par filtration des leucocytes, ledit milieu comprenant plusieurs couches d'un même type qui sont formées d'au moins un matériau non-tissé poreux, dans laquelle au moins une couche a été pressée par calandrage préalablement à son empilement, ladite au moins une couche calandrée étant disposée du coté aval de l'empilement, tandis que le milieu comprend au moins une couche non calandrée.

Selon un deuxième aspect, l'invention propose un système à poches pour la déleucocytation d'un fluide tel que le sang ou un composant sanguin, qui comprend une poche de recueil du filtrat, ladite poche étant reliée, par l'intermédiaire d'une tubulure et au niveau d'un orifice d'entrée, à un orifice de sortie d'une unité de filtration telle que décrite ci-dessus.

D'autres objets et avantages de l'invention apparaîtront au cours de la description qui suit faite en référence aux dessins annexés.

La figure 1 représente, en vue de face, une unité de filtration selon un mode de réalisation de l'invention.

La figure 2 représente de façon schématique et en coupe selon la ligne II-II, l'unité de filtration de la figure 1.

La figure 3 représente, en vue schématique de face, un système à poches pour la déleucocytation d'un fluide tel que le sang ou un composant sanguin, selon un premier mode de réalisation.

La figure 4 représente un système à poches selon une variante du mode de réalisation de la figure 3.

La figure 5 représente, en vue schématique de face, un système à poches pour la déleucocytation stérile et en circuit clos d'un fluide tel que le sang ou un composant sanguin, selon un premier mode de réalisation.

La figure 6 représente, en vue schématique de face, un système à poches pour la déleucocytation stérile et en circuit clos d'un fluide tel que le sang ou un composant sanguin, selon un deuxième mode de réalisation.

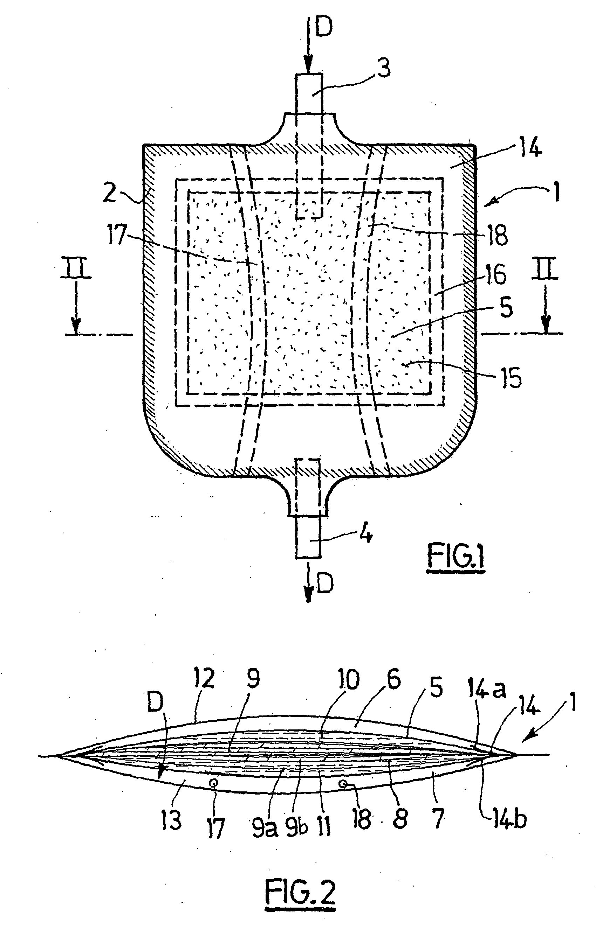

Sur les figures 1 et 2, est représentée une unité de filtration 1 destinée à permettre la déleucocytation d'un fluide tel que le sang ou un composant sanguin. Par composant sanguin, on entend notamment les globules rouges, éventuellement concentrés et/ou en suspension, les plaquettes sanguines, éventuellement concentrées et/ou en suspension, le plasma sanguin, éventuellement pauvre ou riche en plaquettes.

Le sang ou un composant sanguin, après son recueil et sa séparation dans le cas d'un composant, est notamment destiné à être transfusé à un patient qui en a besoin. Lors de cette transfusion, il est bien connu que les leucocytes sont indésirables en ce qu'ils sont susceptibles de provoquer chez le patient des réactions gênantes et/ou potentiellement dangereuses.

C'est pourquoi il est recommandé, voire imposé dans certain pays, de déleucocyter le sang ou le composant sanguin préalablement à sa transfusion, et ce dans un rendement donné. A ce jour, la solution optimale pour éliminer les leucocytes est de filtrer le sang ou le composant sanguin au travers d'une unité de filtration pourvue d'un milieu de déleucocytation.

Dans le mode de réalisation représenté sur les figures 1 et 2, l'unité de filtration 1 comprend une enveloppe extérieure 2 munie d'un orifice d'entrée 3 pour recevoir le fluide à filtrer, et d'un orifice de sortie 4 pour recueillir le filtrat, entre lesquels le fluide à filtrer s'écoule suivant une direction D.

L'unité 1 comprend en outre un élément poreux 5 qui est disposé dans l'enveloppe extérieure 2 de sorte à former un compartiment d'entrée 6 en communication avec l'orifice d'entrée 3 et un compartiment de sortie 7 en communication avec l'orifice de sortie 4.

Dans la description, les termes « entrée », « sortie », « amont » et « aval » sont définis par rapport au sens de circulation du fluide dans l'unité de filtration 1 (voir les flèches D représentées sur les figures 1 et 2).

Lorsque qu'on alimente en fluide l'unité de filtration 1 par l'intermédiaire de l'orifice d'entrée 3, ledit fluide remplit le compartiment d'entrée 6 puis traverse l'élément poreux 5 pour être recueilli dans le compartiment de sortie 7. Ensuite, on peut recueillir le filtrat par l'intermédiaire de l'orifice de sortie 4.

L'élément poreux 5 comprend un milieu de déleucocytation 8 par adsorption et par filtration des leucocytes. Le milieu de déleucocytation 8 comprend plusieurs couches 9 d'un premier type qui sont formées d'au moins un matériau non-tissé poreux. Par type de couches, on entend des couches de matériau ayant sensiblement les mêmes composition, porosité et propriétés physico-chimiques, c'est à dire sensiblement la même capacité de rétention des leucocytes.

Selon une réalisation, les couches 9 peuvent être empilées du coté aval du milieu de déleucocytation 8 suivant la direction d'écoulement D du fluide.

Suivant l'invention, au moins une et pas la totalité de ces couches 9 a été pressée par calandrage, notamment à froid, préalablement à son empilement, la ou les couche(s) calandrée(s) 9a étant disposée(s) du coté aval de l'empilement. L'empilement comprend donc d'amont en aval, au moins une couche non calandrée 9b et au moins une couche calandrée 9a, lesdites couches 9a, 9b étant toutes de même type.

Cette réalisation particulière permet d'obtenir un milieu de déleucocytation 8 dont la capacité d'adsorption et de filtration des leucocytes est améliorée par rapport à un empilement de couches non calandrée. En effet, le calandrage permet notamment de diminuer la porosité moyenne et la perméabilité à l'air de la couche, ce qui augmente sa capacité de rétention des leucocytes. La demanderesse a également constaté qu'en utilisant un milieu de déleucocytation 8 suivant l'invention, le temps entre le prélèvement du fluide et sa filtration pouvait être augmenté sans diminuer sensiblement le taux de déleucocytation, par exemple lorsque ce temps est de 18 heures on obtient encore un taux de déleucocytation satisfaisant.

En outre, par rapport à un empilement de couches toutes calandrées, l'invention permet de limiter les risques de colmatage du milieu de déleucocytation 8 et de maintenir un débit et donc un temps de filtration optimal.

De plus, suivant l'invention, le nombre de couches calandrées 9a peut être ajusté en fonction du rendement de déleucocytation voulu ou imposé par les différentes législations nationales.

Enfin, la solution proposée par l'invention permet de combiner les avantages mentionnés ci-dessus avec une grande simplicité de réalisation de l'empilement puisque les couches calandrées 9a ou non 9b sont de même type.

En variante du mode de réalisation représenté sur les figures 1 et 2, le milieu de déleucocytation 8 peut comprendre en outre au moins une couche d'au moins un deuxième type, la ou lesdites couche(s) étant empilée(s) sur les couches 9 de premier type, du coté amont ou du coté aval à elles.

En particulier, les types de couches peuvent être différents par la nature du matériau qui les forment et/ou par leurs propriétés physico-chimiques.

Suivant une réalisation, la porosité moyenne des couches empilées est décroissante de façon continue ou de façon discrète suivant la direction d'écoulement. Ainsi, il est possible d'optimiser le rendement de déleucocytation en diminuant les risques de colmatage du milieu de déleucocytation 8.

L'élément poreux 5 peut également comprendre un pré-filtre 10 et/ou un post-filtre 11, disposé respectivement coté amont et coté aval du milieu de déleucocytation 8. Le pré-filtre 10 et/ou le post-filtre 11 peuvent être formés d'au moins une couche d'un matériau non-tissé.

Selon une première réalisation, le ou les matériau(x) formant les couches 9 est(sont) hydrophile(s), notamment en cellulose ou ses dérivés, par exemple l'acétate de cellulose.

Selon une deuxième réalisation, le ou les matériau(x) formant les couches 9 est(sont) choisi(s) dans le groupe comprenant les polymères ou les copolymères à base de polypropylène, de polyester, de polyamide, de polyéthylène haute ou basse densité, de polyuréthanne, de fluorure de polyvinylidène, de polyvinylpyrrolidone et leurs dérivés.

Ces produits polymériques ne sont généralement pas hydrophiles naturellement et doivent être traités par des méthodes physiques et/ou chimiques, pour leur conférer lesdites propriétés hydrophiles.

Ces traitements consistent par exemple dans le greffage de substituants hydrophiles, par exemple des groupements de type hydroxyle ou carboxylique, sur le polymère, selon des méthodes connues.

De tels polymères rendus hydrophiles par traitement physique et/ou chimique sont disponibles sur le marché.

On décrit ci-dessous, en relation avec les figures 1 et 2, un mode de réalisation d'une unité de filtration 1.

Dans le mode de réalisation représenté, l'enveloppe extérieure 2 est souple et formée par l'assemblage de deux feuilles 12, 13 de matière plastique souple assemblées mutuellement, par exemple par soudage, sur leur périphérie.

L'élément poreux 5 est maintenu dans l'enveloppe extérieure 2 par des moyens d'association étanches déformables qui sont formés d'un cadre souple 14.

Le cadre souple 14 est formé par un assemblage de deux feuilles 14a, 14b, par exemple plastifiées, entre lesquelles est placé l'élément poreux 5.

Ces deux feuilles 14a, 14b sont ajourées dans leur partie centrale et comportent chacune au moins une ouverture 15 permettant le passage du fluide à filtrer.

Les deux feuilles 14a, 14b sont fixées entre elles de préférence au niveau de la périphérie de l'élément poreux 5, par exemple par un cordon de soudure 16, réalisé à travers l'élément poreux 5, assurant à la fois la fixation de l'élément poreux 5 mais également l'étanchéité.

La soudure des feuilles 14a, 14b à travers l'élément poreux 5 provoque une compression, formant un cordon étanche autour de l'élément poreux 5.

Le cadre souple 14 est soudé sur sa périphérie avec les feuilles extérieures 12, 13 formant l'enveloppe extérieure 2, mutuellement sur tout leur pourtour et au niveau de leur périphérie, assurant ainsi l'étanchéité.

Lors de ce soudage, l'orifice d'entrée 3, formé d'une portion de tubulure, est disposé d'un coté du cadre souple 14 et l'orifice de sortie 4, formé d'une autre portion de tubulure, est disposé de l'autre coté du cadre souple 14.

Ainsi, le compartiment d'entrée 6 formé entre une feuille 12 et l'élément poreux 5 est en communication avec l'orifice d'entrée 3 et le compartiment de sortie 7 formé entre l'autre feuille 13 et l'élément poreux 5 est en communication avec l'orifice de sortie 4.

Pour éviter que l'élément poreux 5 ne se colle contre l'enveloppe extérieure 2, et gène ainsi l'écoulement du fluide, deux joncs d'écartement 17, 18 sont placés à l'intérieur du compartiment de sortie 7, entre l'élément poreux 5 et l'enveloppe extérieure 2.

Ces deux joncs 17, 18 dégagent le compartiment de sortie 7 de l'élément poreux 5 et évitent ainsi que l'élément poreux 5 ne se plaque contre la paroi intérieure de la feuille extérieure 13.

Les joncs 17, 18 peuvent être réalisés à partir de tubulures souples soudées par exemple au niveau de la paroi intérieure de la feuille de l'enveloppe extérieure 2, par exemple au niveau de la soudure périphérique.

Il va de soi que le nombre de joncs d'écartement 17, 18 peut varier, en fonction par exemple des dimensions de l'unité de filtration 1.

Par exemple, il est envisageable de prévoir un jonc d'écartement unique replié de façon à former une boucle à l'intérieur du compartiment de sortie 7.

De préférence, des joncs souples 17, 18 sont utilisés, pour ne pas gêner les possibilités de pliage de l'unité de filtration 1.

Dans un autre mode de réalisation (non représenté), l'enveloppe extérieure 2 est rigide, par exemple réalisée en matériau plastique rigide tel que le polycarbonate.

On donne ci-dessous deux exemples de réalisation d'un élément poreux 5 pour une unité de filtration 1 suivant l'invention.

### Exemple 1

L'élément poreux 5 comprend d'amont en aval et empilées l'une sur l'autre :
- 4 couches de non-tissé en polyester ayant chacune une épaisseur e de l'ordre de 400 µm, une porosité moyenne p = 35 µm et une perméabilité à l'air P comprise entre 1000 et 5000 l/m²/s, en tant que pré-filtre 10;
- 22 couches 9b de non-tissé en polypropylène meltblown ayant chacune 250 µm < e < 400 µm ; 8,5 µm < p < 10 µm et 130 l/m²/s < P < 200 l/m²/s ;
- 2 couches 9a de non-tissé en polypropylène meltblown de même type 9 que les 22 couches 9b précédentes, qui ont été calandrées séparément de sorte à présenter chacune 130 µm < e < 250 µm ; 7 µm < p < 9 µm et 70 l/m²/s < P < 130 l/m²/s;
- 1 couche de non-tissé en polyester meltblown ayant chacune une épaisseur e de l'ordre de 400 µm ; p = 35 µm et 1000 l/m²/s < P < 5000 l/m²/s, en tant que post-filtre 11.

Dans un exemple particulier, cet élément poreux 5 a une surface de filtration comprise entre 50 et 58 cm², par exemple égale à 55 cm², de sorte à permettre la filtration de 450 ml de fluide avec un taux de rétention de 4,8 log (c'est-à-dire que la quantité de leucocytes est divisée par 10^{4,8} en passant au travers de l'élément poreux 5) contre 4,3 avec un élément poreux analogue dans lequel les deux couches 9a n'auraient pas été calandrées, et ce avec un volume mort et un temps de filtration analogues.

Bien entendu, en fonction des objectifs de déleucocytation à atteindre, un nombre de couches 9 différent peut être calandré.

### Exemple 2

L'élément poreux 5 comprend d'amont en aval et empilées l'une sur l'autre :
- 2 couches de non-tissé en polyester ayant chacune une épaisseur e de l'ordre de 400 µm, une porosité moyenne p = 35 µm et une perméabilité à l'air P comprise entre 1000 et 5000 l/m²/s, en tant que pré-filtre 10;
- 2 couches de non-tissé en polypropylène meltblown ayant chacune 250 µm < e < 400 µm ; 10 µm < p < 20 µm et 250 l/m²/s < P < 400 l/m²/s ;
- 18 couches 9b de non-tissé en polypropylène meltblown ayant chacune 250 µm < e < 400 µm ; 8,5 µm < p < 10 µm et 130 l/m²/s < P < 200 l/m²/s ;
- 2 couches 9a de non-tissé en polypropylène meltblown de même type 9 que les 18 couches 9b précédentes, qui ont été calandrées séparément de sorte à présenter chacune 130 µm < e < 250 µm ; 7 µm < p < 9 µm et 70 l/m²/s < P < 130 l/m²/s ;
- 1 couches de non-tissé en polyester meltblown ayant chacune une épaisseur e de l'ordre de 400 µm ; p = 35 µm et 1000 l/m²/s < P < 5000 l/m²/s, en tant que post-filtre 11.

Dans un exemple particulier, cet élément poreux 5 a une surface de filtration comprise entre 15 et 35 cm², par exemple égale à 20 cm², de sorte à permettre la filtration de 200 ml de fluide.

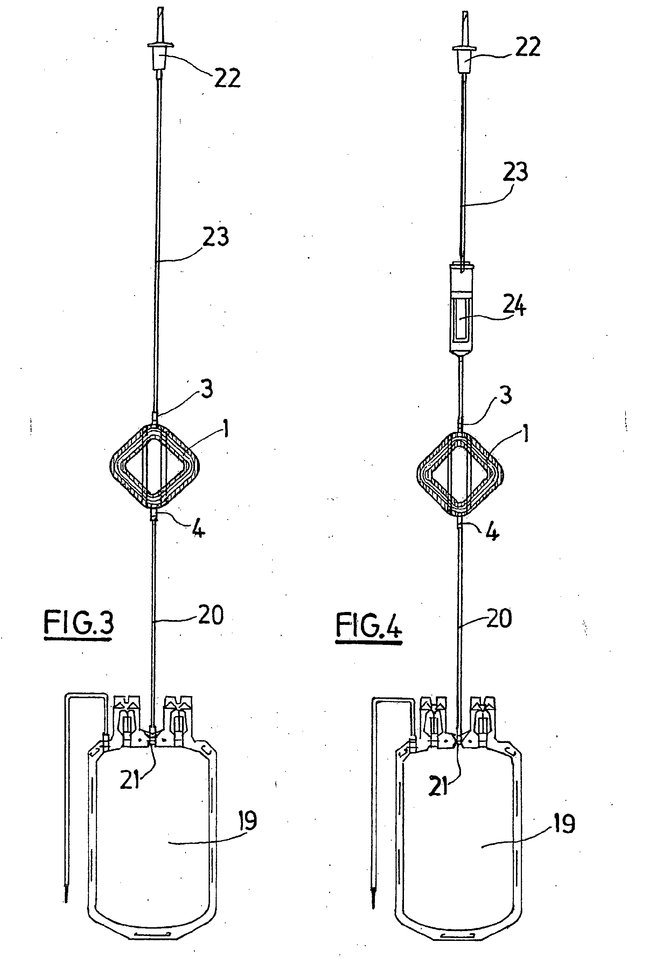

On décrit maintenant, en relation avec les figures 3 et 4, un premier mode de réalisation d'un système à poches pour la déleucocytation d'un fluide tel que le sang ou un composant sanguin qui comprend une poche de recueil 19 du filtrat, ladite poche étant reliée, par l'intermédiaire d'une tubulure 20 et au niveau d'un orifice d'entrée 21, à un orifice de sortie 4 d'une unité de filtration 1 selon l'invention.

Le système comprend en outre des moyens de connexion 22 avec une poche contenant le fluide à filtre qui sont connectés, par l'intermédiaire d'une tubulure 23, à un orifice d'entrée 3 de l'unité de filtration 1.

Ainsi, le fluide, une fois collecté, peut être introduit dans le système à poches pour être filtré au moyen de l'unité de filtration 1, le filtrat étant ensuite recueilli dans la poche 19.

Dans la variante représentée sur la figure 4, un filtre à microagrégats 24 est connecté au système en amont de l'unité de filtration 1.

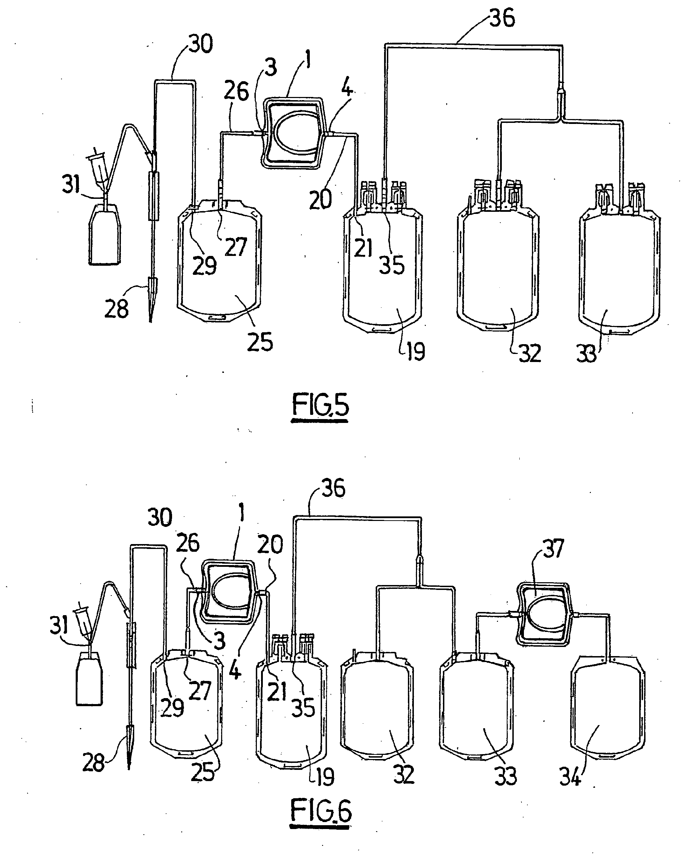

On décrit ci-dessous, en relation avec les figures 5 et 6, un premier et un deuxième mode de réalisation d'un système à poches pour la déleucocytation stérile et en circuit clos d'un fluide tel que le sang ou un composant sanguin, ledit système comprenant une unité de filtration 1 selon l'invention.

A cet effet, les systèmes à poches comprennent une poche de collecte 25 destinée à contenir le fluide à filtrer qui a été préalablement remplie d'une solution de conservation par exemple de type CPD, ladite poche 25 étant reliée par l'intermédiaire d'une tubulure 26 et au niveau de l'un de ses orifices de sortie 27 à l'orifice d'entrée 3 de l'unité de filtration 1 et une poche de recueil 19 destinée à recevoir le filtrat, ladite poche 19 étant reliée par l'intermédiaire d'une tubulure 20 et au niveau de l'un de ses orifices d'entrée 21 à l'orifice de sortie 4 de ladite unité de filtration 1.

Les systèmes à poches comprennent en outre des moyens de prélèvement 28 du sang total connectés à un orifice d'entrée 29 de la poche 25 par l'intermédiaire d'une tubulure 30 munie d'un dispositif 31 de recueil d'un échantillon de sang prélevé.

Les systèmes à poches comprennent également un ensemble de poches satellites 32-34 relié à un orifice de sortie 35 de la poche 19 par l'intermédiaire d'une tubulure 36.

Le système selon le premier mode de réalisation (figure 5) comprend deux poches satellites 32, 33 dont l'une 32 contient une solution de conservation des globules rouges par exemple de type SAGM. Il permet, après sa stérilisation, de réaliser en circuit clos successivement les étapes suivantes :
- recueil du sang total dans la poche de collecte 25 ;
- filtration du sang total ;
- centrifugation de la poche de recueil 19 ;
- recueil des différents constituants du sang dans les poches 19, 33, à savoir d'un concentré de globules rouges additionné de la solution de conservation dans la poche 19 et du plasma dans la poche 33.

Le système selon le deuxième mode de réalisation (figure 6) comprend trois poches satellites 32-34 dont l'une 32 contient une solution de conservation des globules rouges par exemple de type SAGM et une unité de filtration 37 du plasma qui est connectée entre les poches 33, 34. Il permet, après sa stérilisation, de réaliser en circuit clos successivement les étapes suivantes :
- recueil du sang total dans la poche de collecte 25 ;
- filtration du sang total ;
- centrifugation de la poche de recueil 19 ;
- recueil des différents constituants du sang dans les poches 19, 33, à savoir d'un concentré de globules rouges additionné de la solution de conservation dans la poche 19 et du plasma dans la poche 33 ;
- filtration du plasma à travers l'unité de filtration 37 de sorte à éliminer les éléments cellulaires ;
- recueil du plasma filtré dans la poche 34.

En variante, les tubulures sont souples, sécables et soudables afin de pouvoir, après la filtration et avant la centrifugation, dissocier l'unité de filtration 1 du système à poches.

## Revendications

1. Unité de filtration (1) destinée à permettre la déleucocytation d'un fluide tel que le sang ou un composant sanguin, comprenant une enveloppe extérieure (2) munie d'au moins un orifice d'entrée (3) et d'au moins un orifice de sortie (4) entre lesquels le fluide à filtrer s'écoule suivant une direction (D), l'enveloppe (2) renfermant un élément poreux (5) comprenant un milieu de déleucocytation (8) par adsorption et par filtration des leucocytes, ledit milieu comprenant plusieurs couches (9) d'un même type qui sont formées d'au moins un matériau non-tissé poreux, ladite unité étant **caractérisée en ce qu'**au moins une couche (9a) a été pressée par calandrage préalablement à son empilement, ladite au moins une couche calandrée (9a) étant disposée du coté aval de l'empilement, tandis que le milieu (8) comprend au moins une couche non calandrée (9b).

2. Unité de filtration selon la revendication 1, **caractérisée en ce que** les couches (9) sont empilées du coté aval de l'élément poreux (5) suivant la direction d'écoulement (D).

3. Unité de filtration selon la revendication 1 ou 2, **caractérisée en ce que** le milieu de déleucocytation (8) comprend en outre au moins une couche d'au moins un deuxième type, la ou lesdites couche(s) étant empilée(s) sur les couches (9) de premier type, du coté amont ou du coté aval à elles.

4. Unité de filtration selon la revendication 3, **caractérisée en ce que** le premier et le deuxième type de couche sont différents par la nature du matériau qui les forment.

5. Unité de filtration selon la revendication 3 ou 4, **caractérisée en ce que** le premier et le deuxième type de couche sont différents par leurs propriétés physico-chimiques.

6. Unité de filtration selon l'une quelconque des revendications 3 à 5, **caractérisée en ce que** la porosité moyenne des couches empilées est décroissante de façon continue ou de façon discrète suivant la direction d'écoulement (D).

7. Unité de filtration selon l'une quelconque des revendications 1 à 6, **caractérisée en ce que** l'élément poreux (5) comprend en outre un pré-filtre (10) disposé coté amont du milieu de déleucocytation (8).

8. Unité de filtration selon l'une quelconque des revendications 1 à 7, **caractérisée en ce que** l'élément poreux (5) comprend en outre un post-filtre (11) disposé coté aval du milieu de déleucocytation (8).

9. Unité de filtration selon l'une quelconque des revendications 1 à 8, **caractérisée en ce que** le ou les matériau(x) formant les couches est(sont) hydrophile(s).

10. Unité de filtration selon l'une quelconque des revendications 1 à 8, **caractérisée en ce que** le ou les matériau(x) formant les couches est(sont) choisi(s) dans le groupe comprenant les polymères ou les copolymères à base de polypropylène, de polyester, de polyamide, de polyéthylène haute ou basse densité, de polyuréthanne, de fluorure de polyvinylidène, de polyvinylpyrrolidone et leurs dérivés, le ou lesdits matériau(x) étant rendu(s) hydrophile(s) par un traitement physique ou chimique.

11. Unité de filtration selon l'une quelconque des revendications 1 à 10, **caractérisée en ce que** l'enveloppe extérieure (2) est formée de deux feuilles (12, 13) de matière plastique souple assemblées sur leur périphérie.

12. Unité de filtration selon l'une quelconque des revendications 1 à 11, **caractérisée en ce que** l'élément poreux (5) est maintenu dans l'enveloppe extérieure (2) par des moyens d'association étanches déformables.

13. Unité de filtration selon la revendication 12 lorsqu'elle dépend de la revendication 11, **caractérisée en ce que** les moyens d'association comprennent un cadre souple (14) dans lequel l'élément poreux (5) est maintenu, le cadre souple (14) étant formé de deux feuilles souples (14a, 14b) ajourées entre lesquelles l'élément poreux (5) est placé, lesdites feuilles étant fixées d'une part entre elles au niveau de la périphérie de l'élément poreux (5) par un cordon de soudure (16) réalisé à travers l'élément poreux (5) et, d'autre part, au niveau de la périphérie de l'enveloppe extérieure (2) par soudure avec les feuilles (12, 13) formant l'enveloppe extérieure (2).

14. Système à poches pour la déleucocytation d'un fluide tel que le sang ou un composant sanguin, **caractérisé en ce qu'**il comprend une poche de recueil (19) du filtrat, ladite poche étant reliée, par l'intermédiaire d'une tubulure (20) et au niveau d'un orifice d'entrée (21), à un orifice de sortie (4) d'une unité de filtration (1) selon l'une quelconque des revendications 1 à 13.

15. Système à poches selon la revendication 14, **caractérisé en ce qu'**il comprend en outre une poche de collecte (25) destiné à contenir le fluide à filtrer, ladite poche étant reliée, par l'intermédiaire d'une tubulure (26) et au niveau d'un orifice de sortie (27), à un orifice d'entrée (3) de ladite unité de filtration, de sorte à permettre la filtration stérile et en circuit clos du fluide.

16. Système à poches selon la revendication 14 ou 15, **caractérisé en ce qu'**il comprend en outre un ensemble de poches satellites (32-34) relié, par l'intermédiaire d'une tubulure (36), à un orifice de sortie (35) de la poche de recueil (19).

17. Système à poches selon la revendication 16, **caractérisé en ce que** l'ensemble de poches satellites (32-34) comprend au moins deux poches (33, 34) et une autre unité de filtration (37), ladite unité étant disposée de sorte à être ou à pouvoir être mise en communication fluidique avec les deux poches (33, 34) de l'ensemble.

18. Système à poches selon l'une quelconque des revendications 15 à 17, **caractérisé en ce qu'**il comprend en outre des moyens de prélèvement (28) du fluide connectés à un orifice d'entrée (29) de la poche de collecte (25).

## Claims

1. A filtration unit (1) designed to enable leukocytes to be removed from a fluid such as blood or a blood component, said filtration unit comprising an outer casing (2) provided with at least one inlet orifice (3) and with at least one outlet orifice (4) between which the fluid to be filtered flows in one direction (D), said casing enclosing a porous element (5) comprising a leukocyte-removal medium (8) for removing leukocytes by absorbing them and filtering them out, said medium including a plurality of layers (9) of the same type, each of which is formed by at least one porous non-woven material, said unit being **characterized in that** at least one layer (9a) is pressed by calendering prior to being stacked, said at least one calendered layer (9a) being disposed on the downstream side of the stack, while the medium (8) includes at least one non-calendered layer (9b).

2. A filtration unit according to claim 1, **characterized in that** the layers (9) are stacked on the downstream side of the porous element (5) in the flow direction (D).

3. A filtration unit according to claim 1 or claim 2, **characterized in that** the leukocyte-removal medium (8) further includes at least one layer of at least a second type, said layer(s) being stacked on the layers (9) of the first type, on the upstream side or on the downstream side thereof.

4. A filtration unit according to claim 2, **characterized in that** the first and second types of layer differ **in that** they are formed of different types of material.

5. A filtration unit according to claim 3 or claim 4, **characterized in that** the first and second types of layer differ **in that** they have different physico-chemical properties.

6. A filtration unit according to any one of claims 3 to 5, **characterized in that** the mean porosity of the stacked layers decreases continuously or stepwise in the flow direction (D).

7. A filtration unit according to any one of claims 1 to 6, **characterized in that** the porous element (5) further comprises a pre-filter (10) on the upstream side of the leukocyte-removal medium (8).

8. A filtration unit according to any one of claims 1 to 7, **characterized in that** the porous element (5) further comprises a post-filter (11) disposed on the downstream side of the leukocyte-removal medium (8).

9. A filtration unit according to any one of claims 1 to 8, **characterized in that** the material(s) forming the layers is(are) hydrophilic.

10. A filtration unit according to any one of claims 1 to 8, **characterized in that** the material(s) forming the layers is(are) chosen from the group comprising polymers or copolymers based on polypropylene, on polyester, on polyamide, on high-density or low-density polyethylene, on polyurethane, on polyvinylidene fluoride, on polyvinylpyrrolidone, and derivatives thereof, said material(s) being made hydrophilic by physical or chemical treatment.

11. A filtration unit according to any one of claims 1 to 10, **characterized in that** the outer casing (2) is made up of two sheets (12, 13) of flexible plastics material that are assembled together around their peripheries.

12. A filtration unit according to any one of claims 1 to 11, **characterized in that** the porous element (5) is held in the outer casing (2) by deformable impervious association means.

13. A filtration unit according to claim 12, as dependent on claim 11, **characterized in that** the association means comprise a flexible frame (14) in which the porous element (5) is held, the flexible frame (14) being made up of two perforated flexible sheets (14a, 14b) between which the porous element (5) is placed, said sheets being fastened firstly to each other around the periphery of the porous element (5) by means of a welding or heat-sealing bead or seam (16) formed through the porous element (5), and secondly to the outer casing (2) by being welded or heat-sealed to the sheets (12, 13) making up the outer casing (2).

14. A system of bags for removing leukocytes from a fluid such as blood or a blood component, said system being **characterized in that** it comprises a reception bag (19) for collecting the filtrate, said bag being connected, via a connection tube (20) and at an inlet orifice (21), to an outlet orifice (4) of a filtration unit (1) according to any one of claims 1 to 13.

15. A system of bags according to claim 14, **characterized in that** it further comprises a collection bag (25) serving to contain the fluid to be filtered, said bag being connected, via a connection tube (26) and at an outlet orifice (27), to an inlet orifice (3) of said filtration unit, so as to enable the fluid to be filtered under sterile and closed-circuit conditions.

16. A system of bags according to claim 14 or claim 15, **characterized in that** it further comprises a set of satellite bags (32, 34) that is connected, via a connection tube (36) to an outlet orifice (35) of the reception bag (19).

17. A system of bags according to claim 16, **characterized in that** the set of satellite bags (32-34) comprises at least two bags (33, 34) and another filtration unit (37), said unit being disposed so as to be or so as to be capable of being put in fluid communication with the two bags (33, 34) of the set.

18. A system of bags according to any one of claims 15 to 17, **characterized in that** it further comprises tap means (28) for tapping off or taking the fluid, which means are connected to an inlet (29) of the collection bag (25).

## Patentansprüche

1. Filterungseinheit (1), die zur Entfernung von Leukozyten aus einem Fluid, wie z.B. Blut oder einem Blutbestandteil, dienen soll, und eine Außenhülle (2) umfasst, die mit mindestens einer Eintrittsöffnung (3) und mindestens einer Austrittsöffnung (4) versehen ist, zwischen denen das zu filternde Fluid in einer Richtung (D) fließt, wobei die Hülle (2) ein poröses Element (5) umschließt, das ein Medium zur Entfernung von Leukozyten (8) durch Adsorption und Filterung derselben enthält, wobei besagtes Medium mehrere Schichten (9) des gleichen Typs umfasst, die aus mindestens einem porösen Nonwoven-Material gebildet werden, wobei besagte Einheit **dadurch gekennzeichnet ist, dass** zumindest eine Schicht (9a) vor dem Aufschichten durch Kalandrieren zusammengepresst wurde, und zumindest diese eine kalandrierte Schicht (9a) hinter der Aufschichtung angeordnet ist, wohingegen das Medium (8) mindestens eine nicht kalandrierte Schicht (9b) umfasst.

2. Filterungseinheit gemäß Patentanspruch 1, **dadurch gekennzeichnet, dass** die Schichten (9) hinter dem porösen Element (5) einer Fließrichtung folgend aufgeschichtet sind.

3. Filterungseinheit gemäß Patentanspruch 1 oder 2, **dadurch gekennzeichnet, dass** das zur Leukozytenentfernung bestimmte Medium (8) ferner mindestens eine Schicht zumindest eines zweiten Typs umfasst, wobei die Schicht(en) auf den Schichten (9) des ersten Typs davor oder dahinter aufgeschichtet sind.

4. Filterungseinheit gemäß Patentanspruch 3, **dadurch gekennzeichnet, dass** sich der erste Schichttyp vom zweiten durch die Beschaffenheit seines Materials unterscheidet.

5. Filterungseinheit gemäß Patentanspruch 3 oder 4, **dadurch gekennzeichnet, dass** sich der erste Schichttyp vom zweiten durch seine physikalisch-chemischen Eigenschaften unterscheidet.

6. Filterungseinheit gemäß einem der Patentansprüche 3 bis 5, **dadurch gekennzeichnet, dass** die durchschnittliche Porosität der übereinander liegenden Schichten kontinuierlich oder diskontinuierlich in Fließrichtung (D) abnimmt.

7. Filterungseinheit gemäß einem der Patentansprüche 1 bis 6, **dadurch gekennzeichnet, dass** das poröse Element (5) ferner einen Vorfilter (10) umfasst, der vor dem zur Leukozytenentfernung bestimmten Medium (8) angeordnet ist.

8. Filterungseinheit nach einem der Patentansprüche 1 bis 7, **dadurch gekennzeichnet, dass** das poröse Elemente (5) ferner einen Nachfilter (11) umfasst, der hinter dem zur Leukozytenentfernung bestimmten Medium (8) angeordnet ist.

9. Filterungseinheit nach einem der Patentansprüche 1 bis 8, **dadurch gekennzeichnet, dass** das oder die Materialien, die die Schichten bilden, hydrophil ist (sind).

10. Filterungseinheit nach einem der Patentansprüche 1 bis 8, **dadurch gekennzeichnet, dass** das oder die Materialien, die die Schichten bilden, aus der Gruppe der Polymere oder Copolymere aus Polypropylen, Polyester, Polyamid, Polyethylen hoher oder niedriger Dichte, Polyurethan, Polyvinylidenfluorid, Polyvinylpyrrolidon und deren Derivaten ausgewählt wird (werden), wobei das oder die Materialien durch eine physikalische oder chemische Behandlung hydrophil gemacht wird (werden).

11. Filterungseinheit nach einem der Patentansprüche 1 bis 10, **dadurch gekennzeichnet, dass** die Außenhülle (2) durch zwei Folien (12, 13) aus weichem Kunststoff geformt ist, die am Rand miteinander verbunden sind.

12. Filterungseinheit nach einem der Patentansprüche 1 bis 11, **dadurch gekennzeichnet, dass** das poröse Element (5) in der Außenhülle (2) durch undurchlässige verformbare Verbindungsmittel in Position gehalten wird.

13. Filterungseinheit nach Patentanspruche 12, wenn dieser von Patentanspruch 11 abhängt, **dadurch gekennzeichnet, dass** die Verbindungsmittel einen elastischen Rahmen (14) umfassen, in dem das poröse Element (5) in Position gehalten wird, wobei der elastische Rahmen (14) durch zwei durchbrochene weiche Folien (14a, 14b) gebildet wird, zwischen denen das poröse Element (5) positioniert ist, wobei die besagten Folien zum einen am Rand des porösen Elements (5) durch eine durch das poröse Element (5) hindurchführende Schweißnaht (16) miteinander verbunden sind, und zum anderen am Rand der Außenhülle (2) durch Verschweißung mit den Folien (12, 13), die die Außenhülle (2) bilden.

14. Beutelsystem zur Entfernung von Leukozyten aus einem Fluid, wie z.B. Blut oder einem Blutbestandteil, **dadurch gekennzeichnet, dass** es einen Auffangbeutel (19) für das Filtrat umfasst, wobei besagter Beutel über ein Filterrohr (20) in Höhe der Eintrittsöffnung (21) mit einer Austrittsöffnung (4) einer Filterungseinheit (1) gemäß einem der Patentansprüche 1 bis 13 verbunden ist.

15. Beutelsystem nach Patentanspruch 14, **dadurch gekennzeichnet, dass** es ferner einen Sammelbeutel (25) für das Filterfluid umfasst, wobei besagter Beutel über ein Filterrohr (26) in Höhe einer Austrittsöffnung (27) mit einer Eintrittsöffnung (3) besagter Filterungseinheit (1) verbunden ist, so dass eine sterile Filterung im geschlossenen Fluidkreislauf möglich ist.

16. Beutelsystem nach Patentanspruch 14 oder 15, **dadurch gekennzeichnet, dass** es ferner Nebenbeutel (32-34) umfasst, die durch ein Filterohr (36) mit einer Austrittsöffnung (35) des Auffangbeutels (19) verbunden sind.

17. Beutelsystem nach Patentanspruch 16, **dadurch gekennzeichnet, dass** die Nebenbeutel (32-34) mindestens zwei Beutel (33, 34) und eine andere Filterungseinheit (37) umfasst, wobei besagte Einheit so angeordnet ist, dass sie mit den beiden Beuteln (33, 34) der Einheit eine fluidische Verbindung herstellt bzw. herstellen kann.

18. Beutelsystem nach Patentanspruch 15 bis 17, **dadurch gekennzeichnet, dass** es ferner Mittel zur Entnahme (28) des Fluids umfasst, die an eine Eintrittsöffnung (29) des Sammelbeutels (25) angeschlossen sind.
